(19) Europäisches Patentamt
European Patent Office
Office européen des brevets

(11) EP 1 674 075 B1

(12) **FASCICULE DE BREVET EUROPEEN**

(45) Date de publication et mention de la délivrance du brevet:
**20.07.2016 Bulletin 2016/29**

(51) Int Cl.:
*A61Q 5/02* (2006.01) *A61Q 5/10* (2006.01)
*A61K 8/39* (2006.01) *A61K 8/41* (2006.01)
*A61K 8/60* (2006.01)

(21) Numéro de dépôt: **05292743.1**

(22) Date de dépôt: **20.12.2005**

(54) **Procédé de coloration des fibres kératiniques comprenant une étape de lavage**

Verfahren zur Färbung keratinischer Fasern enthaltend einen Haarreinigungsvorgang

Hair colouring process comprising a cleaning step

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **23.12.2004 FR 0453186**

(43) Date de publication de la demande:
**28.06.2006 Bulletin 2006/26**

(73) Titulaire: **L'Oréal**
**75008 Paris (FR)**

(72) Inventeurs:
- **Lalleman, Boris**
  **75015 Paris (FR)**
- **Hercouet, Leïla**
  **93360 Neully Plaisance (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 0 459 900** **EP-A- 0 687 669**
**EP-A- 1 321 131** **WO-A-01/41714**
**WO-A-01/97759** **WO-A-95/22312**
**WO-A-02/058656** **WO-A-02/058659**
**FR-A- 2 362 116** **FR-A- 2 570 598**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet un nouveau procédé de coloration des fibres kératiniques comprenant une étape de coloration et une étape de lavage.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La coloration obtenue à partir de ce type de composition est une coloration permanente. Cependant, elle a tendance à s'affadir au cours du temps, en particulier eu fur et à mesure des shampoings.

Des shampooings peu agressifs ont été développés pour remédier à ce problème de la dégradation de la coloration lors des shampooings. Par exemple, la demande de brevet WO 2001/097759 décrit des shampoings non agressifs à base de tensio-actifs dérivés de sucre choisis parmi les alkyl et alcényl oligoglucoside ou un tensio-actif N-alkylpolyhydroxyalkylamide d'acide gras, ces tensio-actifs étant associés à un glycéride partiel d'acide gras.

Selon ce document, ce shampooing permet d'améliorer la tenue aux lavages des couleurs et d'augmenter l'intensité de la coloration quelque soit le type de coloration mise en oeuvre.

Malgré le développement de ce type de shampooing, le phénomène de dégradation de la couleur lors des shampoings est toujours présent, en particulier sur les cheveux sensibilisés.

Le but de la présente invention est de développer de nouvelles techniques afin de remédier au problème lié à la dégradation de la couleur, shampooing après shampooing, tout en conservant de bonnes qualités d'usages du shampooing.

Ce but est atteint avec la présente invention qui a pour objet un procédé de coloration des fibres kératiniques comprenant une étape de coloration des fibres kératiniques à partir d'une composition de coloration d'oxydation qui contient, dans un milieu approprié à la coloration, au moins une base d'oxydation et au moins un coupleur aromatique substitué en position méta par deux groupements amino dont les deux groupements amino sont primaires, puis une étape de lavage des fibres ainsi colorées avec une composition détergente comprenant au moins un tensio-actif choisi parmi les tensio-actifs alkylpolyglycosides et les tensio-actifs polyglycérolés, l'application de ladite composition détergente étant mise en oeuvre avec un décalage dans le temps d'au moins 12 heures après l'application de la composition de coloration.

Selon l'invention, le coupleur aromatique peut être hétérocyclique ou non.

Dans le cadre de l'invention, on entend par « groupement amino primaire » un groupement -NH2.

Un tel procédé permet de colorer de façon satisfaisante les cheveux tout en limitant la dégradation de la couleur lors des shampoings répétés. Le procédé de l'invention permet aussi de diminuer le phénomène de dégorgement de la couleur et permet aussi de limiter le tâchage de la peau et des tissus.

Un tel procédé est particulièrement efficace sur des cheveux sensibilisés. On entend par cheveux sensibilisés des cheveux ayant par exemple subi des traitements cosmétiques modifiant chimiquement le cheveu tels qu'une permanente, une décoloration, un défrisage ou une combinaison de ces traitements ou par exemple l'action prolongée de la lumière ou l'action mécanique du brossage répété.

Selon l'invention, la base d'oxydation utile dans l'étape de coloration d'oxydation peut notamment être choisie parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide :

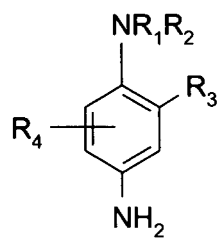

(I)

dans laquelle :

- $R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_2$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_3$ représente un atome d'hydrogène, un atome d'halogène tel qu'un atome de chlore, de brome, d'iode ou de fluor, un radical alkyle en $C_1$-$C_4$, monohydroxyalkyle en $C_1C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
- $R_4$ représente un atome d'hydrogène, d'halogène ou un radical alkyle en $C_1$-$C_4$.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-p-acétylaminoéthyloxy paraphénylènediamine, la N-(β-méthoxyéthyl) paraphénylènediamine, et leurs sels d'addition avec un acide.
Parmi les bases doubles utilisables à titre de bases d'oxydation dans les compositions tinctoriales conformes à l'invention, on peut notamment citer les composés répondant à la formule (II) suivante, et leurs sels d'addition avec un acide :

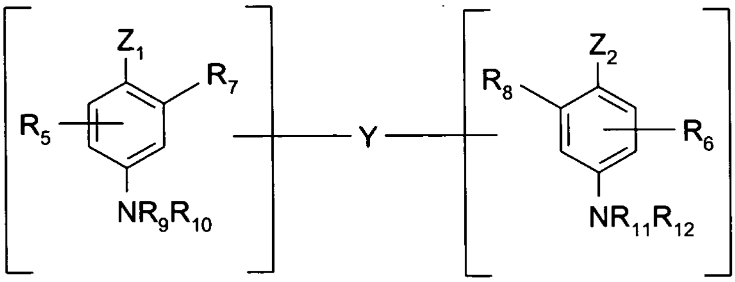 (II)

dans laquelle :

- $Z_1$ et $Z_2$, identiques ou différents, représentent un radical hydroxyle ou -$NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$ $R_{11}$ et $R_{12}$, identiques ou différents, représentent un atome d'hydrogène, un bras de liaison Y ou un radical alkyle en $C_1$-$C_4$ ;

étant entendu que les composés de formule (II) ne comportent qu'un seul bras de liaison Y par molécule.
Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.
Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,6-dioxaoctane, et leurs sels d'addition avec un acide.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide :

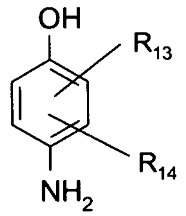

(III)

dans laquelle :

- $R_{13}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{14}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{13}$ ou $R_{14}$ représente un atome d'hydrogène.

A titre de para-aminophénols on peut aussi citer le 4 amino,6 [(5'-amino-2' hydroxy 3'methylphenyl)methyl] 2 méthylphenol et le bis (5 amino 2'hydroxyphenyl méthane et leurs sels d'addition avec un acide.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés diaminopyrazoles tels que les 4,5 diaminopyrazoles ou les 3,4-diaminopyrazoles, notamment ceux décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique :

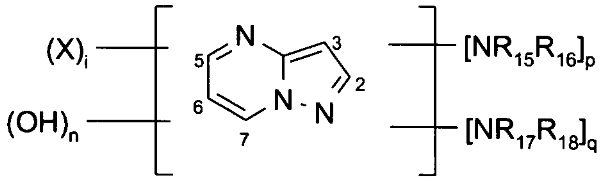

(IV)

dans laquelle :

- $R_{15}$, $R_{16}$, $R_{17}$ et $R_{18}$, identiques ou différents désignent, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical ($C_1$-$C_4$)alcoxy alkyle en $C_1$-$C_4$, un radical aminoalkyle en $C_1$-$C_4$ (l'amine pouvant être protégée par un radical acétyle, uréido ou sulfonyle), un radical ($C_1$-$C_4$)alkylamino alkyle en $C_1$-$C_4$, un radical di-[($C_1$-$C_4$)alkyl] amino alkyle en $C_1$-$C_4$ (les radicaux dialkyles pouvant former un cycle carboné ou un hétérocycle à 5 ou 6 chaînons), un radical hydroxy($C_1$-$C_4$)alkyl- ou di-[hydroxy($C_1$-$C_4$) alkyl]-amino alkyle en $C_1$-$C_4$ ;
- les radicaux X désignent , identiques ou différents, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical amino alkyle en $C_1$-$C_4$, un radical ($C_1$-$C_4$)alkyl amino alkyle en $C_1$-$C_4$, un radical di-[($C_1$-$C_4$)alkyl] amino alkyle en $C_1$-$C_4$ (les dialkyles pouvant former un cycle carboné ou un hétérocycle à 5 ou 6 chaînons), un radical hydroxy($C_1$-$C_4$)alkyl ou di-[hydroxy($C_1$-$C_4$)alkyl]amino alkyle en $C_1$-$C_4$, un radical amino, un radical ($C_1$-$C_4$)alkyl- ou di-[($C_1$-$C_4$)alkyl]-amino ; un atome d'halogène, un groupe acide carboxylique, un groupe acide sulfonique ;
- i vaut 0, 1, 2 ou 3 ; p vaut 0 ou 1 ; q vaut 0 ou 1 ; n vaut 0 ou 1 ; sous réserve que la somme p + q est différente de 0 ;
- lorsque p + q est égal à 2, alors n vaut 0 et les groupes $NR_{15}R_{16}$ et $NR_{17}R_{18}$ occupent les positions (2,3) ; (5,6) ; (6,7) ; (3,5) ou (3,7) ;
- lorsque p + q est égal à 1 alors n vaut 1 et le groupe $NR_{15}R_{16}$ (ou $NR_{17}R_{18}$) et le groupe OH occupent les positions (2,3) ; (5,6) ; (6,7) ; (3,5) ou (3,7) ;

Parmi les pyrazolo-[1,5-a]-pyrimidines de formule (IV) ci-dessus on peut notamment citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol ; le 2-(7-amino pyrazolo-[1,5-a]-pyrimidin-3-ylamino)-éthanol ; le 2-[(3-Amino-pyrazolo[1,5-a]pyrimidin-7-yl)-(2-hydroxy-éthyl)-amino]-éthanol ; le 2-[(7-Amino-pyrazolo[1,5-a]pyrimidin-3-yl)-(2-hydroxy-éthyl)-amino]-éthanol ; la 5,6-diméthyl pyrazolo-[1,5-a]-pyrimidine-3,7-diamine ; la 2,6-diméthyl pyrazolo-[1,5-a]-pyrimidine-3,7-diamine ; la 2, 5, N 7, N 7-tetraméthyl pyrazolo-[1,5-a]-pyrimidine-3,7-diamine ; et leurs sels d'addition et leurs formes tautomères, lorsqu'il existe un équilibre tautomérique.

La ou les bases d'oxydation présentes dans la composition de l'invention sont en général présentes chacune en quantité comprise entre 0,001 et 10 % en poids environ du poids total de la composition tinctoriale, de préférence entre 0,005 et 6 %.

Selon un mode de réalisation particulier, les coupleurs peuvent être substitués uniquement par les deux groupements amino en position méta. Ils peuvent aussi être substitués par un ou plusieurs substituants additionnels, de préférence un ou deux groupements additionnels sur le cycle aromatique.

Le cycle aromatique est par exemple un cycle benzènique, naphtalénique, pyridiniques, pyrimidiniques.

A titre d'exemple de coupleur aromatique hétérocyclique, on peut citer la 2,6-diméthoxypyridine-3,5-diamine et la pyridine-2,6-diamine ou leurs sels d'addition.

Selon un mode de réalisation particulier, le coupleur aromatique utile dans la présente invention est un coupleur méta-phénylène diamine dont les deux groupements amino sont primaires. A ce titre, on peut citer la métaphénylène diamine, le 2,4-diamino phénoxyéthanol, la 4-fluoro-6-methylbenzene-1,3-diamine, le 1,3 bis (2,4-diaminophenoxy)propane, le 1-β-aminoéthyloxy-2,4-diaminobenzène, le 1-β-hydroxyéthyl-2,4-diaminobenzène, l'acide (2,4-diaminophénoxy)acétique, le 4,6-bis (β-hydroxyéthoxy)-1,3-diaminobenzène, le 2,4-diamino 5-méthyléthoxybenzène, le 2,4-diamino 5-β-hydroxyéthyloxytoluène, le 2,4-diméthoxy 1,3-diaminobenzène ou leurs sels d'addition.

La composition de coloration d'oxydation peut en outre contenir un ou plusieurs coupleurs conventionnellement utilisés pour la teinture de fibres kératiniques. Parmi ces coupleurs, on peut notamment citer les méta-phénylènediamines différents des méta-phénylènediamines définies ci dessus, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(ß-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 1,3-bis-(2,4-diaminophénoxy) propane, la 3-uréido aniline, le sésamol, le 1-ß-hydroxyéthylamino-3,4-méthylènedioxybenzène, l'$\alpha$-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, le 1-N-(ß-hydroxyéthyl)amino-3,4-méthylène dioxybenzène, le 2,6-bis-(ß-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

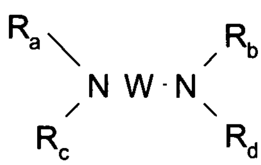
(II)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale utile dans le procédé de l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'étape de coloration d'oxydation est en général mise en oeuvre en présence d'un agent oxydant. Cet agent

oxydant, appliqué sur les fibres kératiniques en présence de la composition de coloration pendant un temps suffisant permet de développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition de coloration d'oxydation qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'étape de lavage du procédé de l'invention est mise en oeuvre à partir d'une composition détergente qui contient un tensio-actif non ionique choisi parmi les tensio-actifs alkylpolyglycosides et les tensio-actifs polyglycérolés.

Les tensio-actifs alkypolyglucosides sont bien connus de la technique et peuvent être plus particulièrement représenté par la formule générale suivante :

$$R_1O\text{-}(R_2O)_t\,(G)_v$$

dans laquelle $R_1$ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, $R_2$ représente un radical alkylène comportant environ de 2 à 4 atomes de carbone, G représente un sucre réduit comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (II) dans laquelle R1 désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (II), peut aller de 1 à 15, de préférence de 1 à 4. Le degré moyen de polymérisation est plus particulièrement compris entre 1 et 2.

Les liaisons glycosidiques entre les motifs sucre sont de type 1-6 ou 1-4, de préférence 1-4.

Des composés de formule (II° sont notamment représentés par les produits vendus par la société COGNIS sous les dénominations PLANTAREN® (600 CS/U, 1200, 2000) ou Plantacare® (818, 1200 et 2000). on peut aussi utiliser les produits vendus par la société SEPPIC sous les dénominations TRITON CG 110 5ou ORAMIX CG 110) et TRITON CG 312 (ou ORAMIX NS 10), les produits vendus par la société BASF sous la dénomination LUTENSOL GB 70 ou encore ceux vendus par la société CHEM Y sous la dénomination AG10 LK.

On peut également utiliser par exemple l'alkyl en C8/C16 polyglucoside 1,4 en solution aqueuse à 53 % commercialisé par COGNIS sous la référence PLANTACARE® 818 UP.

Les tensio-actifs polyglycérolés peuvent être représentés par les composés de formules suivantes :

$$RO[CH_2CH(CH_2OH)O]_mH \text{ ou } RO(CH(CH_2OH)CH_2O]_mH \text{ ou } RO[CH_2CH(OH)CH2O]_mH;$$

dans laquelle R représente un radical hydrocarboné, saturé ou insaturé, linéaire ou ramifié, éventuellement mono ou polyhydroxylé, comportant de 8 à 40 atomes de carbone et de préférence de 10 à 30 atomes de carbone ; m est un nombre préférence compris entre 1 et 10, plus particulièrement de 1,5 à 6.

R peut également comprendre des hétéroatomes tels que par exemple l'oxygène ou l'azote. En particulier, R peut

éventuellement comprendre un ou plusieurs hydroxy et/ou éther et/ou amide.

R désigne de préférence des radicaux alkyle et/ou alcényle en C10-C20, éventuellement mono ou polyhydroxylé.

On peut utiliser par exemple, l'hydroxylauryléther polyclycérolé (3,5 moles) commercialisé sous la dénomination Chimexane® NF de Chimex.

Conformément à un mode de réalisation particulier de l'invention, la teneur totale en tensio-actif non ionique est comprise entre 4 et 50 % en poids par rapport au poids de la composition détergente, de préférence entre 6 et 30 % en poids par rapport au poids de la composition détergente.

La composition détergente peut contenir d'autres tensio-actifs conventionellement utilisés dans les compositions détergentes, par exemple un ou plusieurs tensioactifs non ioniques différents de ceux cités précédemment, un ou plusieurs tensioactifs amphotères ou zwitterioniques, un ou plusieurs tensioactifs anioniques de préférence doux.

Plus particulièrement, à titre de tensioactif non ionique autres que ceux utiles pour l'invention, on peut citer :

- les alcools gras oxyalkylénés;
- les alkylphénols dont la chaîne alkyle est en $C_8$-$C_{18}$ oxyalkylénés ;
- les amides gras oxyalkylénés ou polyglycérolés ;
- les amines grasses oxyalkylénées ;
- les huiles végétales oxyalkylénées ;
- les esters d'acides gras du sorbitan, éventuellement oxyalkylénés ;
- les esters d'acides gras du sucrose, éventuellement oxyalkylénés ;
- les esters d'acides gras du polyéthylèneglycol ;
- les dérivés de N-alkyl glucamine ;
- les oxydes d'amines tels que les oxydes d'alkyl ($C_{10}$ - $C_{14}$) amines ou les oxydes de N-acylaminopropylmorpholine ;
- les copolymères d'oxyde d'éthylène et de propylène.

Par chaîne grasse, on entend une chaîne hydrocarbonée comprenant 6 à 30 atomes de carbone, de préférence de 8 à 24 atomes de carbone, linéaire ou ramifiée, saturée ou non.

Plus particulièrement, le nombre moyen de motifs oxyalkylénés est compris entre 2 et 30 motifs. De préférence, il s'agit de motifs oxyéthylénés, oxypropylénés ou leurs mélanges.

A titre de tensioactifs amphotères ou zwitterioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylampho-dipropionate, Disodium Capryloamphodipropionate, Lauroamphodipropionic acid, Cocoamphodipropionic acid, disodium cocoamphocarboxy ethyl hydroxypropyl sulfonate.

A titre d'exemple on peut citer le cocoamphodiacetate commercialisé sous la dénomination commerciale MIRANOL® C2M concentré par la société Rhodia Chimie.

En ce qui concerne les tensioactifs anioniques doux, on peut citer notamment les composés de formules suivantes, ainsi que leurs mélanges :

- les acides alkyl éther carboxyliques polyoxyalkylénés,
- les acides alkylaryl éther carboxyliques polyoxyalkylénés,
- les acides alkylamido éther carboxyliques polyoxyalkylénés en particulier ceux comportant 2 à 50 groupements oxyde d'éthylène,
- les acides d'alkyl D galactoside uroniques
- les acylsarcosinates, les acylglutamates ;
- les esters d'alkylpolyglycosides carboxyliques ;
- les sels d'acides gras.

Tout particulièrement, on utilise des acides alkyl éther carboxyliques polyoxyalkylénés comme par exemple l'acide lauryl ether carboxylique (4,5 OE) commercialisé par exemple sous la dénomination AKYPO RLM 45 CA de KAO.

Lorsqu'ils sont présents, la teneur en tensioactifs anioniques doux, amphotères et/ou zwittérionique représente de 4 à 50 % en poids par rapport au poids total de la composition détergente.

La composition peut aussi contenir de faibles quantités d'alkyléthersulfates ou d'alkylsulfates. Ces quantités sont de préférence inférieures à 5%.

La composition détergente peut de plus comprendre les additifs classiques dans le domaine comme par exemple ceux choisis parmi la liste non exhaustive tels que les agents réducteurs, les agents oxydants, les séquestrants, les adoucissants, les agents anti-mousse, les agents hydratants, les agents émollients, les agents alcalinisants, les plastifiants, les filtres solaires, les pigments, les charges minérales, les argiles, les minéraux colloïdaux, les nacres, les agents nacrant, les parfums, les peptisants, les conservateurs, les polymères fixant ou non, les protéines, les vitamines, les agents antipelliculaires, les alcools aliphatiques ou aromatiques, et plus particulièrement l'éthanol, l'alcool benzylique, les polyols modifiés ou non tels que le glycérol, le glycol, le propylène glycol, le dipropylène glycol, le butylène glycol, le butyle diglycol, les silicones volatiles, les huiles minérales, organiques ou végétales, les cires oxyéthylénées ou non, les paraffines, les acides gras, les polymères épaississants associatifs ou non, les amides grasses, les esters gras, les alcools gras, les agents conditionneur de préférence des agents conditionneur cationique tels que des polymères cationiques contenant des motifs comportant des groupements amine primaires, secondaires, tertiaires et/ou quaternaires pouvant soit faire partie de la chaîne principale polymère, soit être portés par un substituant latéral directement relié à celle-ci.

Les adjuvants cités ci-dessus sont en général présents en quantité comprise, pour chacun d'eux, entre 0,01 et 20% en poids par rapport au poids de la composition. Les compositions détergentes peuvent se présenter sous différentes formes galéniques tels qu'une lotion, un spray, une mousse aérosol, une pompe mousse, etc.

Le procédé de l'invention peut contenir d'autres étapes, en particulier, il peut contenir des étapes de rinçage des fibres kératiniques, des étapes de conditionnement des fibres kératiniques, des étapes de séchage. Selon un mode de réalisation, le procédé comprend une ou plusieurs étapes intermédiaires de lavage à partir d'une composition détergente comprenant un tensio-actif anionique traditionnel, de préférence choisi parmi les alkyléthersulfates et/ou les alkylsulfates.

De plus, la mise en oeuvre des étapes de coloration d'oxydation et de lavage des fibres kératiniques avec les compositions utiles pour l'invention peut être différée dans le temps, par exemple avec un décalage d'au moins 12 heures entre l'étape de coloration et la ou les étapes de lavages avec la composition détergente de l'invention. On peut aussi mettre en oeuvre plusieurs étapes de lavages à partir de la composition détergente utile dans l'invention pour une seule étape de coloration.

Selon un mode de réalisation particulier, les fibres kératiniques colorés sont des fibres kératiniques sensibilisées.

## EXEMPLES

Exemple 1 :

Les compositions de teintures suivantes ont été réalisées à partir des différents coupleurs méta-phénylènediamine décrits ci dessous

| | | |
|---|---|---|
| 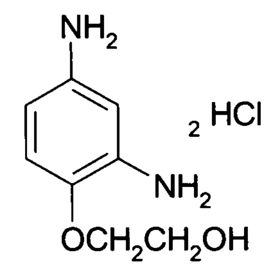 | 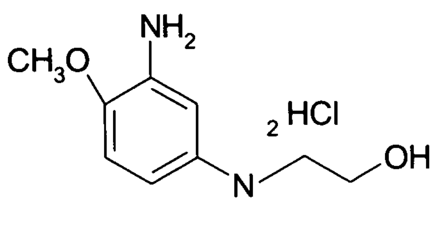 | 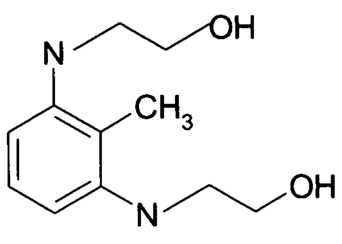 |
| **2,4 diamino phénoxyéthanol 2 HCl** | **1-méthoxy-2-amino-4-(2'-hydroxy-éthylamino) benzène 2 HCl** | **2,6-bis-(-(2-hydroxy-éthylamino)-1-méthyl benzène** |

| | **Composition 1 (Invention)** | **Composition 2 (hors invention)** | **Composition 3 Comparatif)** |
|---|---|---|---|
| Décyl glucoside en solution aqueuse à 60% d'Oramix® CG 110 (SEPPIC) | 5.4g | 5.4g | 5.4g |
| Alcool éthylique 96 degrés dénaturé | 18g | 18g | 18g |

(suite)

| | Composition 1 (Invention) | Composition 2 (hors invention) | Composition 3 Comparatif) |
|---|---|---|---|
| Alcool benzylique | 1,8g | 1,8g | 1,8g |
| Polyéthylène glycol (8 OE) | 2,7g | 2,7g | 2,7g |
| Pentasodium pentetate à 40% dans l'eau | 1,08g | 1,08g | 1,08g |
| Sodium Métabisulfite | 0,205g | 0,205g | 0.205g |
| 2,4 diamino phenoxyéthanol, HCl | $6.10^{-3}$ mol | - | - |
| 1-méthoxy-2-amino-4-(2'-hydroxy-éthylamino) benzène, 2 HCl | - | $6.10^{-3}$ mol | - |
| 2,6-bis-(-(2-hydroxy-éthylamino)-1-méthyl benzène | - | - | $6.10^{-3}$ mol |
| para-PhénylèneDiamine | $6.10^{-3}$ mol | $6.10^{-3}$ mol | $6.10^{-3}$ mol |
| Ammoniaque à 20.5% | 10g | 10g | 10g |
| Eau déminéralisée | Qsp 100 | Qsp 100 | Qsp 100 |

Au moment de l'emploi, chacune des compositions ci-dessus est mélangée avec de l'eau oxygénée (Eau oxygénée l'Oréal professionnel 20 volumes à 6%) poids pour poids.
Le mélange est ensuite appliqué sur des mèches de cheveux naturels à 90% blancs fortement décolorées et permanentées, à raison de 10 g de mélange colorant/ g de mèche. Le temps de pause est de 15 minutes de chaque côté de la mèche.
La coloration est ensuite rincée à l'eau puis lavée avec un shampooing commercial (shampooing DOP camomille). Les mèches sont ensuite séchées 30 minutes à 60°C au casque.

Etapes de lavage :

48 heures après la réalisation des colorations précédemment décrites, les mèches subissent des épreuves de lavages avec une composition détergente contenant un tensioactif non ionique tel que décrit dans le tableau 3 :

Tableau 3

| | Composition détergente 1 |
|---|---|
| Coco-glucoside à 53% dans l'eau (Plantacare® 818 UP-Cognis) | 28,30g |
| Acide citrique | qsp pH=6 |
| Eau | qsp 100 |

Trois shampooings sont réalisés, les mèches étant séchées au casque (30 minutes à 60°C) entre chaque shampooing.
Avant le lavage et après les 3 étapes de lavage, la coloration des mèches est évaluée visuellement et par la mesure des valeurs (L*, a*, b*) à l'aide du spectro-colorimètre MINOLTA CM 2022.
La dégradation de la coloration avant et après les trois étapes de lavage au moyen de la composition détergente 1 est exprimée en ΔE selon la formule suivante :

$$\Delta E_{i(\text{après lavage – avant lavage})} = \sqrt{(\Delta L_i^{*2} + \Delta a_i^{*2} + \Delta b_i^{*2})}$$

**Résultats :**

Tableau 4

| Coloration | Composition 1 (inv) | Composition 2 (hors invention) | Composition 3 (comparatif) |
|---|---|---|---|
| Dégradation de la coloration (□E) | 4.7 | 9 | 31 |

Ces résultats montrent une très nette amélioration de la tenue de la coloration lorsque l'on applique la composition détergente 1 sur des mèches colorées par des coupleurs d'oxydation méta-phénylènediamine les deux groupements amino sont primaires.

Il est également constaté que le dégorgement de la couleur dans l'eau et dans la mousse est moins important dans le cas de la présente invention.

**Revendications**

**1.** Procédé de coloration des fibres kératiniques comprenant

- une étape de coloration des fibres kératiniques à partir d'une composition de coloration d'oxydation qui contient, dans un milieu approprié, au moins une base d'oxydation et au moins un coupleur aromatique substitué en position méta par deux groupements amino dont les deux groupements amino sont des groupements amino primaires, puis
- une étape de lavage des fibres ainsi colorées avec une composition détergente comprenant au moins un tensio-actif choisi parmi les tensio-actifs alkylpolyglycosides et les tensio-actifs polyglycérolés, **caractérisé en ce que** l'application de la composition détergente contenant au moins un tensio-actif alkylpolyglycoside ou polyglycérolé est mise en oeuvre avec un décalage dans le temps d'au moins 12 heures après l'application de la composition de coloration.

**2.** Procédé selon la revendication 1 dans lequel la ou les bases d'oxydation sont choisis parmi les paraphénylènediamines, les bases doubles, les para-aminophénols, les ortho aminophénols et les bases d'oxydation hétérocycliques.

**3.** Procédé selon la revendication 1 ou 2 dans lequel la ou les bases d'oxydation sont présentes chacune en quantité comprise entre 0,001 et 10% en poids par rapport au poids de la composition de coloration.

**4.** Procédé selon l'une quelconque des revendications précédentes dans lequel le coupleur est un composé méta-phénylène diamine.

**5.** Procédé selon l'une quelconque des revendications précédentes dans lequel le ou les coupleurs méta-phénylènediamine sont choisis parmi la métaphénylène diamine, le 2,4-diamino phénoxyéthanol, la 4-fluoro-6-methylbenzene-1,3-diamine, le 1,3 bis (2,4-diaminophenoxy)propane, le 1-β-aminoéthyloxy-2,4-diaminobenzène, le 1-β-hydroxyéthyl-2,4-diaminobenzène, l'acide (2,4-diaminophénoxy)acétique, le 4,6-bis (β-hydroxyéthoxy)-1,3-diaminobenzène, le 2,4-diamino 5-méthyléthoxybenzène, le 2,4-diamino 5-β-hydroxyéthyloxytoluène, le 2,4-diméthoxy 1,3-diaminobenzène ou leurs sels d'addition.

**6.** Procédé selon l'une des revendications 1 à 4, **caractérisé en ce que** le ou les coupleurs sont présents chacun en quantité comprise entre 0,001 et 10% en poids par rapport au poids de la composition de coloration.

**7.** Procédé selon l'une quelconque des revendications précédentes, **caractérisé en ce que** la composition de coloration d'oxydation comprend au moins un agent oxydant.

**8.** Procédé selon la revendication précédente, **caractérisée en ce que** l'agent oxydant est choisi parmi le peroxyde d'hydrogène, le peroxyde d'urée, les bromates ou ferricyanures de métaux alcalins, les persels, les enzymes d'oxydoréduction.

**9.** Procédé selon l'une des revendications précédentes, **caractérisé en ce que** la composition de coloration contient un colorant direct.

**10.** Procédé selon l'une quelconque des revendications précédentes, dans lequel le ou les tensio-actifs alkylpolyglycosides répondent à la formule $R_1O-(R_2O)_t (G)_v$ dans laquelle $R_1$ représente un radical alkyle et/ou alcényle linéaire

ou ramifié comportant environ de 8 à 24 atomes de carbone, un radical alkylphényle dont le radical alkyle linéaire ou ramifié comporte de 8 à 24 atomes de carbone, $R_2$ représente un radical alkylène comportant environ de 2 à 4 atomes de carbone, G représente un sucre réduit comportant de 5 à 6 atomes de carbone, t désigne une valeur allant de 0 à 10 et v désigne une valeur allant de 1 à 15.

**11.** Procédé selon l'une quelconque des revendications précédentes dans lequel le ou les tensio-actifs polyglycérolés répondent aux formules $RO[CH_2CH(CH_2OH)O]_mH$ ou $RO[CH(CH_2OH)CH_2O]_mH$ ou $RO[CH_2CH(OH)CH20]_mH$; dans laquelle R représente un radical hydrocarboné, saturé ou insaturé, linéaire ou ramifié, éventuellement mono ou polyhydroxylé, comportant de 8 à 40 atomes de carbone et de préférence de 10 à 30 atomes de carbone ; m est un nombre préférence compris entre 1 et 10, plus particulièrement de 1,5 à 6.

**12.** Procédé selon l'une quelconque des revendications précédentes dans lequel la teneur totale en tensioactif non ionique est comprise entre 4 et 50 % en poids par rapport au poids de la composition détergente

**13.** Procédé selon l'une quelconque des revendications précédentes dans lequel la composition détergente comprend de plus un ou plusieurs tensio-actif non ioniques différent des tensio-actifs alkylpolyglycosides et des tensio-actifs polyglycérolés, un ou plusieurs tensio-actifs amphotères, un ou plusieurs tensio-actifs zwitterioniques, un ou plusieurs tensio-actifs anionique doux.

**14.** Procédé selon la revendication 12 dans lequel la composition détergente comprend un ou plusieurs tensio-actifs choisis parmi :

- les alcools gras oxyalkylénés;
- les alkylphénols dont la chaîne alkyle est en $C_8$-$C_{18}$, oxyalkylénés ;
- les amides gras oxyalkylénés ou polyglycérolés ;
- les amines grasses oxyalkylénées ;
- les huiles végétales oxyalkylénées ;
- les esters d'acides gras du sorbitan, éventuellement oxyalkylénés ;
- les esters d'acides gras du sucrose, éventuellement oxyalkylénés ;
- les esters d'acides gras du polyéthylèneglycol ;
- les dérivés de N-alkyl glucamine ;
- les oxydes d'amines tels que les oxydes d'alkyl ($C_{10}$ - $C_{14}$) amines ou les oxydes de N-acylaminopropylmorpholine ;
- les copolymères d'oxyde d'éthylène et de propylène.

**15.** Procédé selon l'une quelconque des revendications précédentes, **caractérisée en ce que** la composition détergente comprend au moins un agent conditionneur, de préférence cationique.

**16.** Procédé selon l'une quelconque des revendications précédentes dans lequel la composition détergente contient un tensio-actif alkylsulfate et/ou alkyléthersulfate.

**17.** Procédé selon la revendication 16 dans lequel la quantité d'alkyléthersulfate et/ou alkylsulfate est inférieure à 5 %.

**18.** Procédé selon l'une quelconque des revendications précédentes comprenant une étape de lavage intermédiaire au moyen d'une composition détergente au moyen d'une composition détergente comprenant un tensio-actif anionique choisi parmi les alkyléther et/ou les alkylsulfates.

**19.** Procédé selon l'une quelconque des revendications précédentes comprenant une ou plusieurs étapes supplémentaires de rinçage, de lavage, de conditionnement, de séchage.

**20.** Procédé selon l'une quelconque des revendications précédentes comprenant plusieurs étapes de lavage à partir de la composition détergente telle que définie à l'une des revendications 1 à 17.

**21.** Procédé selon l'une quelconque des revendications précédentes pour le traitement de fibres kératiniques sensibilisées.

## Patentansprüche

**1.** Verfahren zum Färben von Keratinfasern, umfassend

• einen Schritt des Färbens der Keratinfasern ausgehend von einer Oxidationsfärbezusammensetzung, die in einem geeigneten Medium mindestens eine Oxidationsbase und mindestens einen aromatischen Kuppler, der in meta-Position durch zwei Aminogruppen substituiert ist, wobei es sich bei den beiden Aminogruppen um primäre Aminogruppen handelt, enthält, und dann
• einen Schritt des Waschens der so gefärbten Fasern mit einer Reinigungszusammensetzung, die mindestens ein Tensid, das aus Alkylpolyglycosid-Tensiden und Polyglycerin-Tensiden ausgewählt ist, umfasst, **dadurch gekennzeichnet, dass** die Anwendung der mindestens ein Alkylpolyglycosid- oder Polyglycerin-Tensid enthaltenden Reinigungszusammensetzung mit einer Zeitverzögerung von mindestens 12 Stunden nach dem Aufbringen der Färbezusammensetzung durchgeführt wird.

**2.** Verfahren nach Anspruch 1, bei dem die Oxidationsbase bzw. die Oxidationsbasen aus paraPhenylendiaminen, Doppelbasen, para-Aminophenolen, ortho-Aminophenolen und heterocyclischen Oxidationsbasen ausgewählt wird bzw. werden.

**3.** Verfahren nach Anspruch 1 oder 2, bei dem die Oxidationsbase bzw. die Oxidationsbasen jeweils in einer Menge zwischen 0,001 und 10 Ges.-%, bezogen auf das Gewicht der Färbezusammensetzung, vorliegt bzw. vorliegen.

**4.** Verfahren nach einem der vorhergehenden Ansprüche, wobei es sich bei dem Kuppler um eine meta-Phenylendiaminverbindung handelt.

**5.** Verfahren nach einem der vorhergehenden Ansprüche, bei dem der meta-Phenylendiamin-Kuppler bzw. die meta-Phenylendiamin-Kuppler aus meta-Phenylendiamin, 2,4-Diaminophenoxyethanol, 4-Fluor-6-methylbenzol-1,3-diamin, 1,3-Bis(2,4-diaminophenoxy)propan, 1-β-Aminoethyloxy-2,4-diaminobenzol, 1-β-Hydroxyethyl-2,4-diaminobenzol, (2,4-Diaminophenoxy)essigsäure, 4,6-Bis(β-hydroxyethoxy)-1,3-diaminobenzol, 2,4-Diamino-5-methylethoxybenzol, 2,4-Diamino-5-β-hydroxyethyloxytoluol und 2,4-Dimethoxy-1,3-diaminobenzol oder Additionssalzen davon ausgewählt ist bzw. sind.

**6.** Verfahren nach einem der Ansprüche 1 bis 4, **dadurch gekennzeichnet, dass** der Kuppler bzw. die Kuppler jeweils in einer Menge zwischen 0,001 und 10 Gew.-%, bezogen auf das Gewicht der Färbezusammensetzung, vorliegt bzw. vorliegen.

**7.** Verfahren nach einem der vorhergehenden Ansprüche, **dadurch gekennzeichnet, dass** die Oxidationsfärbezusammensetzung mindestens ein Oxidationsmittel umfasst.

**8.** Verfahren nach dem vorhergehenden Anspruch, **dadurch gekennzeichnet, dass** das Oxidationsmittel aus Wasserstoffperoxid, Harnstoffperoxid, Alkalimetallbromaten oder -ferricyaniden, Persalzen, Redoxenzymen ausgewählt wird.

**9.** Verfahren nach einem der vorhergehenden Ansprüche, **dadurch gekennzeichnet, dass** die Färbezusammensetzung einen Direktfarbstoff enthält.

**10.** Verfahren nach einem der vorhergehenden Ansprüche, bei dem das Alkylpolyglycosid-Tensid bzw. die Alkylpolyglycosid-Tenside der Formel $R_1O\text{-}(R_2O)_t(G)_v$ entspricht bzw. entsprechen, wobei $R_1$ für einen linearen oder verzweigten Alkyl- und/oder Alkenylrest mit etwa 8 bis 24 Kohlenstoffatomen oder einen Alkylphenylrest, in dem der lineare oder verzweigte Alkylrest 8 bis 24 Kohlenstoffatome enthält, steht, $R_2$ für einen Alkylenrest mit etwa 2 bis 4 Kohlenstoffatomen steht, G für einen reduzierten Zucker mit 5 bis 6 Kohlenstoffatomen steht, t für einen Wert im Bereich von 0 bis 10 steht und v für einen Wert im Bereich von 1 bis 15 steht.

**11.** Verfahren nach einem der vorhergehenden Ansprüche, bei dem das Polyglycerin-Tensid bzw. die Polyglycerin-Tenside den Formeln $RO[CH_2CH(CH_2OH)O]_mH$ oder $RO[CH(CH_2OH)CH_2O]_mH$ oder $RO[CH_2CH(OH)CH_2O]_mH$ entspricht bzw. entsprechen; wobei R für einen linearen oder verzweigten, gesättigten oder ungesättigten, gegebenenfalls mono- oder polyhydroxylierten Kohlenwasserstoffrest mit 8 bis 40 Kohlenstoffatomen und vorzugsweise 10 bis 30 Kohlenstoffatomen steht; m für eine Zahl steht, die vorzugsweise zwischen 1 und 10 liegt und spezieller 1,5 bis 6 beträgt.

**12.** Verfahren nach einem der vorhergehenden Ansprüche, bei dem der Gesamtgehalt an nichtionischem Tensid zwischen 4 und 50 Gew.-%, bezogen auf das Gewicht der Reinigungszusammensetzung, liegt.

**13.** Verfahren nach einem der vorhergehenden Ansprüche, bei dem die Reinigungszusammensetzung außerdem ein oder mehrere nichtionische Tenside, die von den Alkylpolyglycosid-Tensiden und Polyglycerin-Tensiden verschieden sind, ein oder mehrere amphotere Tenside, ein oder mehrere zwitterionische Tenside, ein oder mehrere milde anionische Tenside umfasst.

**14.** Verfahren nach Anspruch 12, bei dem die Reinigungszusammensetzung ein oder mehrere aus:

- oxyalkylenierten Fettalkoholen;
- oxyalkylenierten Alkylphenolen mit $C_8$-$C_{18}$-Alkylkette;
- oxyalkylenierten oder polyglycerinierten Fettamiden;
- oxyalkylenierten Fettaminen;
- oxyalkylenierten Pflanzenölen;
- gegebenenfalls oxyalkylenierten Sorbitanfettsäureestern;
- gegebenenfalls oxyalkylenierten Saccharosefettsäureestern;
- Polyethylenglykolfettsäureestern;
- N-Alkylglucaminderivaten;
- Aminoxiden wie ($C_{10}$-$C_{14}$) -Alkylaminoxiden oder N-Acylaminopropylmorpholinoxiden;
- Copolymeren von Ethylenoxid und Propylenoxid ausgewählte Tenside umfasst.

**15.** Verfahren nach einem der vorhergehenden Ansprüche, **dadurch gekennzeichnet, dass** die Reinigungszusammensetzung mindestens ein Konditionierungsmittel, das vorzugsweise kationisch ist, umfasst.

**16.** Verfahren nach einem der vorhergehenden Ansprüche, bei dem die Reinigungszusammensetzung ein Alkylsulfat- und/oder Alkylethersulfat-Tensid umfasst.

**17.** Verfahren nach Anspruch 16, bei dem die Menge an Alkylethersulfat und/oder Alkylsulfat weniger als 5% beträgt.

**18.** Verfahren nach einem der vorhergehenden Ansprüche, umfassend einen zwischengeschalteten Waschschritt unter Verwendung einer Reinigungszusammensetzung, die ein aus Alkylethern und/oder Alkylsulfaten ausgewähltes anionisches Tensid umfasst.

**19.** Verfahren nach einem der vorhergehenden Ansprüche, umfassend einen oder mehrere zusätzliche Spül-, Wasch-, Konditionierungs-, Trocknungsschritte.

**20.** Verfahren nach einem der vorhergehenden Ansprüche, umfassend mehrere Waschschritte ausgehend von der Reinigungszusammensetzung gemäß einem der Ansprüche 1 bis 17.

**21.** Verfahren nach einem der vorhergehenden Ansprüche zur Behandlung von sensibilisierten Keratinfasern.

## Claims

**1.** Process for dyeing keratin fibres, comprising

- a step of dyeing the keratin fibres using an oxidation dye composition which contains, in a suitable medium, at least one oxidation base and at least one aromatic coupler substituted in the meta position with two amino groups, the two amino groups being primary amino groups, and then
- a step of washing the fibres thus dyed, with a detergent composition comprising at least one surfactant chosen from alkylpolyglycoside surfactants and polyglycerol surfactants, **characterized in that** the application of the detergent composition containing at least one alkylpolyglycoside or polyglycerol surfactant is performed with a time delay of at least 12 hours after the application of the dye composition.

**2.** Process according to Claim 1, in which the oxidation base(s) are chosen from paraphenylenediamines, double bases, para-aminophenols, ortho-aminophenols and heterocyclic oxidation bases.

**3.** Process according to Claim 1 or 2, in which the oxidation base(s) are each present in an amount of between 0.001% and 10% by weight relative to the weight of the dye composition.

**4.** Process according to any one of the preceding claims, in which the coupler is a meta-phenylenediamine compound.

**5.** Process according to any one of the preceding claims, in which the meta-phenylenediamine coupler(s) are chosen from meta-phenylenediamine, 2,4-diaminophenoxyethanol, 4-fluoro-6-methylbenzene-1,3-diamine, 1,3-bis(2,4-diaminophenoxy)propane, 1-β-aminoethyloxy-2,4-diaminobenzene, 1-β-hydroxyethyl-2,4-diaminobenzene, (2,4-diaminophenoxy)acetic acid, 4,6-bis(β-hydroxyethoxy)-1,3-diaminobenzene, 2,4-diamino-5-methylethoxybenzene, 2,4-diamino-5-β-hydroxyethyloxytoluene and 2,4-dimethoxy-1,3-diaminobenzene, or addition salts thereof.

**6.** Process according to one of Claims 1 to 4, **characterized in that** the coupler(s) are each present in an amount of between 0.001% and 10% by weight relative to the weight of the dye composition.

**7.** Process according to any one of the preceding claims, **characterized in that** the oxidation dye composition comprises at least one oxidizing agent.

**8.** Process according to the preceding claim, **characterized in that** the oxidizing agent is chosen from hydrogen peroxide, urea peroxide, alkali metal bromates or ferricyanides, persalts and redox enzymes.

**9.** Process according to one of the preceding claims, **characterized in that** the dye composition contains a direct dye.

**10.** Process according to any one of the preceding claims, in which the alkylpolyglycoside surfactant(s) correspond to the formula $R_1O-(R_2O)_t(G)_v$ in which $R_1$ represents a linear or branched alkyl and/or alkenyl radical comprising from about 8 to 24 carbon atoms, an alkylphenyl radical in which the linear or branched alkyl radical comprises from 8 to 24 carbon atoms, $R_2$ represents an alkylene radical comprising from about 2 to 4 carbon atoms, G represents a reduced sugar comprising from 5 to 6 carbon atoms, t denotes a value ranging from 0 to 10 and v denotes a value ranging from 1 to 15.

**11.** Process according to any one of the preceding claims, in which the polyglycerol surfactant(s) correspond to the formulae $RO[CH_2CH(CH_2OH)O]_mH$ or $RO(CH(CH_2OH)CH_2O]_mH$ or $RO(CH_2CH(OH)CH_2O]_mH$; in which R represents a linear or branched, saturated or unsaturated, optionally mono- or polyhydroxylated hydrocarbon-based radical, comprising from 8 to 40 carbon atoms and preferably from 10 to 30 carbon atoms; m is a number preferably between 1 and 10, more particularly from 1.5 to 6.

**12.** Process according to any one of the preceding claims, in which the total content of nonionic surfactant is between 4% and 50% by weight relative to the weight of the detergent composition.

**13.** Process according to any one of the preceding claims, in which the detergent composition also comprises one or more nonionic surfactants other than the alkylpolyglycoside surfactants and the polyglycerol surfactants, one or more amphoteric surfactants, one or more zwitterionic surfactants, and one or more mild anionic surfactants.

**14.** Process according to Claim 12, in which the detergent composition comprises one or more surfactants chosen from:

- oxyalkylenated fatty alcohols;
- oxyalkylenated alkylphenols in which the alkyl chain is $C_8$-$C_{18}$;
- oxyalkylenated or polyglycerolated fatty amides;
- oxyalkylenated fatty amines;
- oxyalkylenated plant oils;
- optionally oxyalkylenated fatty acid esters of sorbitan;
- optionally oxyalkylenated fatty acid esters of sucrose;
- fatty acid esters of polyethylene glycol;
- N-alkylglucamine derivatives;
- amine oxides such as ($C_{10}$-$C_{14}$) alkylamine oxides or N-acylaminopropylmorpholine oxides;
- copolymers of ethylene oxide and of propylene oxide.

**15.** Process according to any one of the preceding claims, **characterized in that** the detergent composition comprises at least one conditioning agent, which is preferably cationic.

**16.** Process according to any one of the preceding claims, in which the detergent composition contains an alkyl sulfate and/or alkyl ether sulfate surfactant.

**17.** Process according to Claim 16, in which the amount of alkyl ether sulfate and/or alkyl sulfate is less than 5%.

**18.** Process according to any one of the preceding claims, comprising an intermediate washing step using a detergent composition comprising an anionic surfactant chosen from alkyl ethers and/or alkyl sulfates.

**19.** Process according to any one of the preceding claims, comprising one or more additional steps of rinsing, washing, conditioning and drying.

**20.** Process according to any one of the preceding claims, comprising several washing steps using the detergent composition as defined in one of Claims 1 to 17.

**21.** Process according to any one of the preceding claims, for treating sensitized keratin fibres.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2001097759 A
- GB 1026978 A
- GB 1153196 A
- DE 2359399
- JP 63169571 A
- JP 3333495 A
- WO 9615765 A
- DE 3843892
- DE 4133957
- WO 9408969 A
- WO 9408970 A
- FR 2733749 A
- DE 19543988
- US 2528378 A
- US 2781354 A